# EUROPÄISCHE PATENTSCHRIFT

(11) **EP 0 391 322 B1**
(45) Veröffentlichungstag und Bekanntmachung des Hinweises auf die Patenterteilung: **08.09.1993**
(21) Anmeldenummer: 90106315.6
(22) Anmeldetag: 03.04.1990
(51) Int. Cl.: A61K 7/043

(54) **Wässriger Nagellack**
Aqueous nail enamel
Vernis à ongles aqueux

(30) Priorität: 07.04.1989 DE 3911262
(43) Veröffentlichungstag der Anmeldung: 10.10.1990
(73) Patentinhaber: rotring international GmbH & Co KG, 22525 Hamburg (DE)
(72) Erfinder: Koch, Detlef, D-2000 Hamburg 71 (DE); Rassek, Richard, F-2050 Hamburg 80 (DE)
(74) Vertreter: UEXKÜLL & STOLBERG

(56) Entgegenhaltungen:
- EP-A- 0 000 819
- EP-A- 0 143 480
- EP-A- 0 170 000
- US-A- 3 422 185

## Beschreibung

Die Erfindung betrifft einen wäßrigen Nagellack.

Die bekannten Nagellacke auf Basis organischer Lösungsmittel haben den Nachteil, daß bei häufiger Anwendung die Lösungsmittel den Nagel angreifen und schädigen können. Außerdem werden bei der Anwendung und Trocknung freigesetzte Lösungsmitteldämpfe eingeatmet, was gesundheitlich nicht unbedenklich ist. Schließlich ist es auch im Hinblick auf die Schonung der Umwelt wünschenswert, organische Lösungsmittel zu vermeiden, wo immer dies möglich ist. Aus all diesen Gründen besteht das Bedürfnis, die bekannten lösungsmittelhaltigen Nagellacke durch wäßrige Nagellacke zu ersetzen.

In der DE-A-27 57 773 ist eine Nagelüberzugszubereitung beschrieben, die aus einem wäßrigen Emulsionspolymerisat besteht. Die Herstellung erfolgt durch wäßrige Emulsionspolymerisation von zwei oder mehr Monomeren, die aus Acrylat-, Methacrylat- oder Styrolmonomeren ausgewählt sind. Dabei muß wenigstens eines der ausgewählten Monomere ein Acrylat- oder Methacrylatmonomeres sein.

Aus der DE-B-28 54 337 ist eine kosmetische Zubereitung zur Verstärkung der Nägel bekannt. Gemäß einer Ausführungsform enthält diese in wäßriger Lösung Trimethylolamin, mindestens ein nicht-kationisches kosmetisches Harz und/oder mindestens ein kationisches kosmetisches Harz, mindestens einen Weichmacher und eine anorganische oder anorganische Säure. Bei diese Zusammensetzung handelt es sich nicht um einen Nagellack.

In der DE-A-32 47 172 ist ein tintenförmiger Nagellack beschrieben. Dieser besteht aus einem alkalilöslichem Polyesterharz, Ammoniak, einem kosmetisch zugelassenen sauren organischen Farbstoff, Additiven und Wasser.

Aus der EP-A-0 143 480 sind wäßrige Nagellacke bekannt, welche Polyurethan, Acrylharze und Färbemittel enthalten und welche ohne Hilfsmittel als Film vom Nagel wieder abgezogen werden können.

Diese wenigen bekannten Zusammensetzungen vermögen den Ansprüchen der Verbraucher nicht in jeder Hinsicht zu genügen.

Aufgabe der Erfindung ist es daher, einen weiteren wäßrigen Nagellack mit verbesserten Eigenschaften zu schaffen.

Die Aufgabe wird durch einen wäßrigen Nagellack gelöst, der aus 12 bis 50 Gew.% eines Polyurethans und/oder eines Polyurethancopolymers in dispergierter Form als Bindemittel, 0,1 bis 1 Gew.% Verdicker, Wasser und 2 bis 15 Gew.% eines Acrylat-Styrol-Copolymerisats mit einem Molekular gewicht oberhalb von 200 000 und einer Säurezahl im Bereich von 50 bis 65 sowie gegebenenfalls weiteren Zusatzstoffen besteht.

Erfindungsgemäß geeignete Polyurethane und Polyurethancopolymere sind als wäßrige, feindisperse Kunststoffdispersionen im Handel erhältlich. Die Handelsprodukte haben typischerweise einen Feststoffgehalt von 30 bis 50 Gew.%. Ausgehend von solchen Handelsprodukten kann der Bindemittelgehalt des gewünschten wäßrigen Nagellacks durch Verdünnen mit Wasser eingestellt werden. Erfindungsgemäß besonders bevorzugt ist eine wäßrige Dispersion auf Basis von linearem Polyurethan. Als Polyurethancopolymer ist ein Polyurethan-Acrylat-Copolymer bevorzugt.

Als weiteren notwendigen Bestandteil enthält der erfindungsgemäße wäßrige Nagellack 0,1 bis 1 Gew.% Verdicker. Geeignete Verdickungsmittel sind beispielsweise Naturgummen, wie Guar, Gummiarabikum, Zellulose und Zellulosederivate, Silikate, Tone und synthetische Polymerisate wie Acrylate. Als Ton-Verdicker ist beispielsweise hydrophiler Montmorillonit geeignet, der unter dem Namen Bentone (eingetragenes Warenzeichen) erhältlich ist. Erfindungsgemäß bevorzugt sind Acrylatverdicker, insbesondere Acrylatcopolymerisate.

Die Eigenschaften des erfindungsgemäßen wäßrigen Nagellacks können durch Zusatz eines oder mehrerer wasserlöslicher, natürlicher und/oder synthetischer Harze wesentlich verbessert werden. Die Harze bewirken eine größere Härte der getrockneten Nagellackschicht und außerdem eine bessere Verankerung der Schicht auf dem Nagel. Beispiel für kosmetisch geeignete natürliche Harze sind Kautschuk und Schellack. Beispiele für erfindungsgemäß geeignete synthetische Harze sind Acrylharze, Styrolharze, Vinylharze und Acrylstyrolharze. Das erfindungsgemäß eingesetzte Harz ist ein Acryl-Styrol-Copolymerisat mit einem Molekulargewicht oberhalb von 200 000 und einer Säurezahl im Bereich von 50 bis 65. Die Harze werden vorzugsweise in einer Menge von 2 bis 15 Gew.%, insbesondere 5 bis 12 Gew.% und am meisten bevorzugt 10 bis 12 Gew.%, bezogen auf das Gewicht des Nagellackes, eingesetzt.

In den meisten Fällen ist in dem erfindungsgemäßen Nagellack ein Farbmittel enthalten. Eine große Zahl der in lösungsmittelhaltigen Nagellacken verwendeten Farbmittel sind auch erfindungsgemäß einsetzbar. Die Auswahl der Farbmittel kann daher in der bekannten Weise erfolgen, wobei aber zu beachten ist, daß das Farbmittel wasserverträglich sein muß. Die Auswahl des Farbmittels erfolgt in Abhängigkeit davon, ob ein transparenter, ein opaker oder ein irisierender Nagellack hergestellt werden soll. Es können organische Farbstoffe und Pigmente, anorganische Pigmente, Perlglanz und Fischsilber verwendet werden. Fertigungstechnisch besonders zweckmäßig ist der Einsatz des Farbmittels in Form einer Dispersion, beispielsweise Pigmentdispersion. Auch hinsichtlich der benötigten Mengen an Farbmittel bestehen zu den bekannten lösungsmittelhaltigen Nagellacken keine Unterschiede.

In einigen Fällen kann es zweckmäßig sein, dem erfindungsgemäßen Nagellack ein oder mehrere kosmetisch geeignete Tenside zuzusetzen, und zwar in Mengen bis zu 1 Gew.%.

Die Anwendung des erfindungsgemäßen Nagellacks wird erleichtert, wenn man einen Trocknungsbeschleuniger zusetzt. Geeignete Trocknungsbeschleuniger sind einerseits schwer flüchtige Flüssigkeiten, die gute Lösungsmittel für das Bindemittel sind und deshalb Hautbildung an der Oberfläche der trocknenden Lackschicht vermeiden, und andererseits leicht flüchtige Flüssigkeiten, welche zusammen mit dem Wasser verdampfen. Ein erfindungsgemäß bevorzugter Trocknungsbeschleuniger der ersten Art ist ein schwer flüchtiger Glykolester, insbesondere Butylglykolacetat. Erfindungsgemäß bevorzugte Trocknungsbeschleuniger der zweiten Art sind flüchtige Alkohole, insbesondere Ethanol und/oder 2-Propanol.

Die Trocknungsbeschleuniger werden vorzugsweise in Mengen von 0,1 bis 5 Gew.%, insbesondere 0,5 bis 1 Gew.%, bezogen auf das Gewicht des Nagellacks, eingesetzt.

Zur Erhöhung der Kratzfestigkeit und zur Vermeidung von Oberflächenstörungen bzw. zur Erhöhung der Oberflächenglätte der Lackschicht können dem erfindungsgemäßen Nagellack modifizierte Silikone zugesetzt werden. Geeignet ist beispielsweise ein polyethermodifiziertes Dimethylpolysiloxan-Copolymer. Alternativ können für den gleichen Zweck auch Hartwachse verwendet werden. Diese sind als wäßrige Dispersion im Handel erhältlich, und sie werden zur Herstellung des erfindungsgemäßen Nagellacks vorzugsweise in dieser Form verwendet.

Es kann weiterhin zweckmäßig sein, ein Additiv zuzusetzen, das der Verhinderung der Sedimentation der Bestandteile des Nagellacks, insbesondere der Pigmente, dient. Ein Beispiel für einen erfindungsgemäß geeigneten Sedimentationsverhinderer ist Calciumsilikathydrat.

Natürlich kann der erfindungsgemäße Nagellack auch nagelpflegende und nagelschützende Substanzen enthalten. Beispiele dafür sind Proteine, Vitamine und Sonnenschutzmittel.

Durch den Zusatz von Duftstoffen kann der Nagellack anwendungsfreundlicher gestaltet werden. Schließlich kann durch Zusatz von Konservierungsstoffen die Lagerbeständigkeit erhöht werden.

Die Erfindung betrifft auch die Verwendung einer wäßrigen Polyurethan- und/oder Polyurethancopolymer-Dispersion, gegebenenfalls mit Zusatzstoffen, als Nagellack.

Im folgenden wird die Erfindung anhand von Beispielen näher erläutert.

In der vorliegenden Beschreibung, in den Ansprüchen und in den Beispielen beziehen sich alle Angaben in Gew.% auf das Gesamtgewicht des Nagellacks. Soweit Bestandteile des Nagellacks als Dispersion oder Lösung eingesetzt werden oder eingesetzt werden können, wie beispielsweise Bindemittel oder Farbmittel, so beziehen sich die Angaben in Gew.% auf den damit erzielten Feststoffgehalt im fertigen Nagellack.

### Beispiel 1: Cremelack

- 36,1 Gew.%: Polyurethan-Acrylat-Copolymer als feindisperse wäßrige Dispersion
- 0,45 Gew.%: Montmorillonit-Verdicker (Bentone LT, eingetragenes Warenzeichen)
- 0,5 Gew.%: Calciumsilikathydrat Ca₆Si₆O₁₇ (OH)₂
- 0,06 Gew.%: Acrylatverdicker
- 0,5 Gew.%: Butylglykolacetat
- 0,8 Gew.%: Pigment White 77891 (Flexonylweiß RL-LA, eingetragenes Warenzeichen)
- 0,2 Gew.%: Pigment Red 12355 (Unispers Rot RBS-Pi, eingetragenes Warenzeichen)
- 61,39 Gew.%: Wasser

Das Polyurethan-Acrylat-Copolymer wurde als feindisperse wäßrige Dispersion vorgelegt und unter intensivem Rühren mit dem Montmorillonit-Verdicker und dem Calciumsilikathydrat vermischt. Nach Zugabe des Acrylat-Verdickers und des Butylglykolacetats wurde langsam weitergerührt, bis die Masse hochviskos wurde. Anschließend wurden unter Rühren die Pigmentpräparationen eingearbeitet.

Es wurde ein roter Cremelack mit guter Eignung als Nagellack erhalten.

### Beispiel 2: Perllack

Beispiel 1 wurde mit dem Unterschied wiederholt, daß anstelle der dort verwendeten Pigmente die folgenden Farbmittel eingerührt wurden:
- 1 Gew.%: Pigment White 77019 (Iriodin 120, eingetragenes Warenzeichen) als Perlglanzpigment
- 0,2 Gew.%: Pigment Red 12355 (Unispers Rot RBS-Pi, eingetragenes Warenzeichen)

Es wurde ein roter Perllack mit guten Anwendungseigenschaften als Nagellack erhalten.

### Beispiel 3

- 27,2 Gew.%: Polyurethan als feindisperse wäßrige Dispersion
- 13,8 Gew.%: Acrylcopolymer als wäßrige Dispersion
- 0,08 Gew.%: Acrylat-Verdicker
- 0,5 Gew.%: Butylglykolacetat
- 0,4 Gew.%: Pigment Red 12355 (Unispers Rot RBS-Pi, eingetragenes Warenzeichen)
- 58,02 Gew.%: Wasser

Das Polyurethan wurde als feindisperse wäßrige Dispersion vorgelegt. Das Acrylcopolymer wurde als wäßrige Dispersion unter Rühren hinzugefügt. Anschließend wurde unter Rühren der Acrylatverdicker zugegeben. Es wurde weiter gerührt, bis die Masse hochviskos wurde. Schließlich wurde das Farbmittel eingerührt.

Es wurde ein roter Nagellack mit guten Anwendungseigenschaften erhalten.

## Patentansprüche

1. Wäßriger Nagellack, bestehend aus 12 bis 50 Gew.% eines Polyurethans und/oder eines Polyurethan-Copolymers in dispergierter Form als Bindemittel, 0,1 bis 1 Gew.% Verdicker und Wasser sowie gegebenenfalls weiteren Zusatzstoffen, **dadurch gekennzeichnet,** daß er 2 bis 15 Gew.% Acrylat-Styrol-Copolymerisat mit einem Molekulargewicht oberhalb von 200 000 und einer Säurezahl im Bereich von 50 bis 65 enthält.

2. Wäßriger Nagellack nach Anspruch 1, **dadurch gekennzeichnet,** daß das dispergierte Polyurethan-Copolymer ein Polyurethan-Acrylat-Copolymer ist.

3. Wäßriger Nagellack nach Anspruch 1 oder 2, **dadurch gekennzeichnet,** daß der Verdicker ein Acrylat-Verdicker ist.

4. Wäßriger Nagellack nach Anspruch 1 oder 2, **dadurch gekennzeichnet,** daß der Verdicker ein hydrophiler Montmorillonit-Verdicker ist.

5. Wäßriger Nagellack nach einem der vorhergehenden Ansprüche, **dadurch gekennzeichnet,** daß er als Trocknungsbeschleuniger ein schwer flüchtiges Lösungsmittel für das Bindemittel enthält.

6. Wäßriger Nagellack nach Anspruch 5, **dadurch gekennzeichnet,** daß er als Trocknungsbeschleuniger 0,1 bis 5 Gew.% eines schwer flüchtigen Glykolesters enthält.

7. Wäßriger Nagellack nach Anspruch 6, **dadurch gekennzeichnet,** daß er als Trocknungsbeschleuniger Butylglykolacetat enthält.

8. Wäßriger Nagellack nach einem der Ansprüche 1 bis 4, **dadurch** **gekennzeichnet,** daß er als Trocknungsbeschleuniger 0,1 bis 5 Gew.% eines flüchtigen Alkohols enthält.

9. Wäßriger Nagellack nach Anspruch 8, **dadurch gekennzeichnet,** daß er als Trocknungsbeschleuniger Ethanol und/oder 2-Propanol enthält.

10. Wäßriger Nagellack nach einem der vorhergehenden Ansprüche, **dadurch gekennzeichnet,** daß er ein Farbmittel, ein Netzmittel, als Mittel zur Verbesserung der Kratzfestigkeit ein modifiziertes Silikon, einen Sedimentationsverhinderer, als kosmetische Zusätze Proteine, Vitamine und Sonnenschutzmittel und/oder Konservierungsmittel enthält.

11. Wäßriger Nagellack nach Anspruch 10, **dadurch gekennzeichnet,** daß er als Sedimentationsverhinderer Calciumsilikathydrat enthält.

## Claims

1. An aqueous nail varnish, consisting of 12 to 50% by weight of a polyurethane and(or of a polyurethane copolymer in dispersed form as a binder, 0.1 to 1% by weight thickener and water and also optionally further additives, characterised in that it contains 2 to 15% by weight acrylate-styrene copolymer having a molecular weight above 200,000 and an acid number in the range of 50 to 65.

2. An aqueous nail varnish according to Claim 1, characterised in that the dispersed polyurethane copolymer is a polyurethane-acrylate copolymer.

3. An aqueous nail varnish according to Claim 1 or 2, characterised in that the thickener is an acrylate thickener.

4. An aqueous nail varnish according to Claim 1 or 2, characterised in that the thickener is a hydrophilic montmorillonite thickener.

5. An aqueous nail varnish according to one of the preceding Claims, characterised in that it contains a sparingly volatile solvent for the binder as a drying accelerator.

6. An aqueous nail varnish according to Claim 5, characterised in that it contains 0.1 to 5% by weight of a sparingly volatile glycol ester as a drying accelerator.

7. An aqueous nail varnish according to Claim 6, characterised in that it contains butyl glycol acetate as a drying accelerator.

8. An aqueous nail varnish according to one of Claims 1 to 4, characterised in that it contains 0.1 to 5% by weight of a volatile alcohol as a drying accelerator.

9. An aqueous nail varnish according to Claim 8, characterised in that it contains ethanol and/or 2-propanol as a drying accelerator.

10. An aqueous nail varnish according to one of the preceding Claims, characterised in that it contains a colourant, a cross-linking agent, as agent for improving the scratch-resistance a modified silicone, a sedimentation-preventer, as cosmetic additives proteins, vitamins and sun-protection agents and(or preservatives.

11. An aqueous nail varnish according to Claim 10, characterised in that it contains calcium silicate hydrate as sedimentation-preventer.

## Revendications

1. Vernis à ongles aqueux formé de 12 à 50 % en poids d'un polyuréthane et/ou d'un copolymère de polyuréthane sous forme dispersée en tant qu'agent liant ; 0,1 à 1 % en poids d'agent épaississant et de l'eau ainsi que le cas échéant d'autres substances d'addition, caractérisé en ce qu'il renferme de 2 à 15 % en poids d'un copolymère acrylate/styrène ayant un poids moléculaire de 200 000 et un indice d'acide dans la zone allant de 50 à 65.

2. Vernis à ongles selon la revendication 1, caractérisé en ce que le copolymère de polyuréthane dispersé est un copolymère polyuréthane/acrylate.

3. Vernis à ongles aqueux selon la revendication 1 ou la revendication 2, caractérisé en ce que l'agent épaississant est un épaississant acrylique.

4. Vernis à ongles aqueux selon la revendication 1 ou la revendication 2, caractérisé en ce que l'épaississant est un épaississant à base de montmorillonite hydrophile.

5. Vernis à ongles aqueux selon l'une des revendications précédentes, caractérisé en ce qu'il contient comme accélérateur de séchage un solvant difficilement volatil - pour l'agent liant

6. Vernis à ongles aqueux selon la revendication 5, caractérisé en ce qu'il contient comme accélérateur de séchage de 0,1 à 5 % en poids d'un ester de glycol difficilement volatil.

7. Vernis à ongles aqueux selon la revendication 6, caractérisé en ce qu'il contient comme accélérateur de séchage de l'acétate de butylglycol.

8. Vernis à ongles aqueux selon l'une des revendications 1 à 4, caractérisé en ce qu'il contient comme accélérateur de séchage de 0,1 à 5 % d'un alcool volatil.

9. Vernis à ongles selon la revendication 8, caractérisé en ce qu'en tant qu'accélérateur de séchage, il contient de l'éthanol et/ou du 2-propanol.

10. Vernis à ongles aqueux selon l'une des revendications précédentes, caractérisé en ce qu'il renferme un colorant, un agent de réticulation, en tant qu'agent améliorant la résistance au grattage, une silicone modifiée, un agent empêchant la sédimentation, comme additifs cosmétiques des protéines, des vitamines et des agents protecteurs contre le soleil et/ou des agents conservateurs.

11. Vernis à ongles aqueux selon la revendication 10, caractérisé en ce qu'il renferme comme agent qui empêche la sédimentation, un silicate de calcium hydraté.
